# EUROPEAN PATENT APPLICATION

(11) **EP 1 738 658 A1**
(43) Date of publication of application: **03.01.2007**
(21) Application number: 05720819.1
(22) Date of filing: 15.03.2005
(51) Int. Cl.: A23L 1/30, A61K 31/05, A61K 31/353, A61K 31/7004, A61P 3/10, A61P 43/00

(54) **ACEROLA LEAF EXTRACT-CONTAINING BLOOD SUGAR LEVEL INCREASE INHIBITOR AND AGE FORMATION INHIBITOR, AND FOOD CONTAINING THEM**

(30) Priority: 19.03.2004 JP 2004080520
(71) Applicant: Nichirei Foods Inc., Tokyo 104-8402 (JP)
(72) Inventor: AOKI, Hitoshi, Nichirei Corporation, Chiba-shi Chiba 261-8545 (JP); HANAMURA, Takayuki, Nichirei Corporation, Chiba-shi Chiba 261-8545 (JP); MAYAMA, Chisato, Nichirei Corporation, Chiba-shi Chiba 261-8545 (JP)
(74) Representative: Woods, Geoffrey Corlett
(86) International application number: PCT/JP2005/004564
(87) International publication number: WO 2005/089785

(57) **Abstract**

This invention is directed to providing an inhibitor of blood glucose level elevation and an inhibitor of AGE generation made from natural ingredients and a food product comprising either thereof. Such inhibitor of blood glucose level elevation or inhibitor of AGE generation comprises, as an active ingredient, an acerola leaf extract and/or a processed product thereof.

## Description

### Technical Field

The present invention relates to an inhibitor of blood glucose level elevation and an inhibitor of advanced glycation end product (AGE) generation.

### Background Art

In accordance with changes in dietary habits and lifestyle of recent years, the number of diabetic patients is increasing. At present, the number of diabetic patients is as high as 7,000,000 in Japan, and such number could be as large as 15,000,000 when future diabetics are added.

Diabetes is a metabolic disorder in which a prolonged hyperglycemic state caused by an insufficient level of insulin hormone activities is exhibited. A prolonged hyperglycemic state may result in development of various types of complications, such as nervous disorders, cataract, renal disorders, retinopathy, arthrosclerosis, atherosclerosis, and diabetic gangrene. Complications are predominantly classified as vascular disorders resulting from nonenzymatic glycation whereby a protein binds to a glucose in the blood of a patient and nervous disorders resulting from the destruction of cells due to accumulation of sorbitol generated upon glucose metabolization. A glycated protein formed upon protein-glucose binding produces a compound referred to as an advanced glycation end product (AGE) as the reaction further proceeds. It is deduced that AGE binds to a specific receptor (RAGE) in vascular endothelial cells and contributes to the development of diabetic vascular disorders.

Thus, inhibition of blood glucose level elevation and inhibition of AGE generation are regarded as methods of treating and preventing diabetes and diabetic complications.

In the past, many medical agents for treating and preventing diabetes and diabetic complications have been developed.

Examples of such medical agents include an inhibitor of blood glucose level elevation that is to be administered to a diabetic patient who experiences an abnormal blood glucose level after a meal and an inhibitor of α-glucosidase that inhibits digestion and absorption of carbohydrates to prevent the blood glucose level from becoming elevated. Voglibose and Acarbose are known as representative inhibitors of α-glucosidase. A carbonyl reagent, aminoguanidine, is known as an example of an inhibitor of AGE generation. Such agents have attracted attention as anti-diabetic agents or anti-diabetic complication agents, and a wide variety of clinical experiments have been conducted.

While these agents have remarkable effects, they impose various side effects on patients, such as a bloating sensation upon ingestion, induction of a hypoglycemic state due to the combined use thereof with other hypoglycemic agents, and nausea or headache.

In order to overcome such drawbacks, agents made from natural ingredients, which have mild effects but are free from problems related to side effects, have been developed. For example, a perilla extract (JP Patent Publication (Unexamined) No. 2000-102383), a yerba mate extract (JP Patent Publication (Unexamined) No. 2003-146900), an extract of Apocynum venetum leaves (JP Patent Publication (Unexamined) No. 2002-053486), and a Folium eriobotryae extract (JP Patent Publication (Unexamined) No. 2003-128571), are known as inhibitors of α-glucosidase made from natural ingredients. As inhibitors of AGE generation made from natural ingredients, for example, active anti-diabetic substances and active anti-diabetic complication substances obtained via extraction from rice such as wild rice and a method for producing the same (JP Patent No. 3,334,016) are known, although the number of such agents is not sufficiently large.

Acerola is a tropical fruit of the genus *Malpighia* of the family *Malpighiaceae* of the order *Rutales,* which is native to Caribbean Islands. At present, acerola fruit is used for beverages and health food products throughout the world. However, useful components of acerola leaves have not yet been examined. Acerola leaves are unused resources, and discovery of industrial value thereof has been awaited.

### Disclosure of the Invention

The present invention is directed to providing an inhibitor of blood glucose level elevation and an inhibitor of AGE generation made from natural ingredients and a food product comprising either thereof.

The present invention includes the following inventions.
(1) An inhibitor of blood glucose level elevation comprising, as an active ingredient, an acerola leaf extract and/or a processed product thereof.
(2) The inhibitor of blood glucose level elevation according to (1), wherein the acerola leaf extract and/or the processed product thereof comprise(s) polyphenols.
(3) An inhibitor of AGE generation comprising, as an active ingredient, an acerola leaf extract and/or a processed product thereof.
(4) The inhibitor of AGE generation according to (3), wherein the acerola leaf extract and/or the processed product thereof comprise(s) polyphenols.
(5) The inhibitor of blood glucose level elevation or the inhibitor of AGE generation according to any of (1) to (4), which is used to treat or prevent diabetes or diabetic complications.
(6) The inhibitor of blood glucose level elevation or the inhibitor of AGE generation according to any of (1) to (5), which is to be added to a food product.
(7) A food product comprising the inhibitor of blood glucose level elevation or the inhibitor of AGE generation according to any of (1) to (6).
(8) A food product comprising an acerola leaf extract and/or a processed product thereof.

This description includes part or all of the contents as disclosed in the description and/or drawings of Japanese Patent Application No. 2004-080520, which is a priority document of the present application.

### Effects of the Invention

The present invention provides an inhibitor of blood glucose level elevation and an inhibitor of AGE generation comprising, as an active ingredient, a naturally occurring substance, and a food product comprising either thereof.

### Brief Description of Drawings

Fig. 1 shows the results of a comparison of maltase inhibitory activity measurements of the purified acerola leaf extract prepared in Example 1 and a purified acerola fruit extract.
Fig. 2 shows the results of a measurement of inhibitory activity against AGE generation of the purified acerola leaf extract prepared in Example 1.

### Preferred Embodiments of the Invention

The present invention relates to an inhibitor of blood glucose level elevation and an inhibitor of AGE generation comprising, as an active ingredient, an acerola leaf extract and/or a processed product thereof. The area of production or the variety of acerola from which the acerola leaves used in the present invention are extracted is not particularly limited. For example, acerola can be produced in Okinawa, Japan, or in Brazil. The inhibitor of blood glucose level elevation and the inhibitor of AGE generation of the present invention comprise, as an active ingredient, a naturally-occurring substance, i.e., an acerola leaf extract and/or a processed product thereof. Accordingly, such inhibitors involve few side effects. The present invention involves the effective use of unused resources, i.e., acerola leaves, which renders the present invention preferable.

An acerola leaf extract may be obtained from a fresh, semidried, or dried acerola leaf. Also, an extract may be obtained from a leaf remaining untreated or an adequately ground or shredded leaf.

An acerola leaf extract is not particularly limited as long as it is extracted by a conventional technique. An acerola leaf extract may be concentrated according to need. In the present invention, a processed product of an acerola leaf extract can also be used. The term "a processed product of an acerola leaf extract" used herein includes, but is not limited to, purification products of an acerola leaf extract obtained by various chromatography techniques.

An acerola leaf extract or a processed product thereof is preferably obtained with the use of water or a hydrophilic organic solvent. As a hydrophilic organic solvent, for example, known organic solvents, such as alcohols, such as methyl alcohol, ethyl alcohol, glycerin, propylene glycol, and 1,3-butylene glycol, acetone, tetrahydrofuran, acetonitrile, 1,4-dioxane, pyridine, dimethyl sulfoxide, N,N-dimethylformamide, and acetic acid, can be used. Such hydrophilic organic solvents are preferably mixed with water.

The conditions for extraction are not particularly limited. The temperature range is generally between 0°C and 120°C, and preferably between 20°C and 50°C. The duration of extraction is about 1 to 24 hours, and preferably about 1 or 2 hours. The amount of the solvent used for extraction is preferably 1 to 20 times that of the raw material in terms of mass.

After the completion of the extraction, the residue may be removed via filtration or centrifugation to obtain an extract. An extract may be further concentrated according to need.

The resulting extract occasionally contains saccharides, organic acids, or the like. Accordingly, it is preferable to carry out a step of purification in order to remove such substances. Purification may be carried out via, for example, normal-phase or reverse-phase chromatography, ion-exchange chromatography, or gel filtration. These techniques may be carried out in adequate combination.

As described in the Examples, an acerola leaf extract and/or a processed product thereof used in the present invention has activities of inhibiting blood glucose level elevation and inhibiting AGE generation. Such activities may result from the fact that the acerola leaf extract and/or the processed product thereof comprise(s) polyphenols. Although the compositions of the polyphenols are unknown, such compositions are considered to be effective in terms of giving rise to activities of inhibiting blood glucose level elevation and inhibiting AGE generation.

The content of the acerola leaf extract and/or the processed product thereof in the inhibitor of blood glucose level elevation or the inhibitor of AGE generation of the present invention is not particularly limited as long as desired effects are attained. The inhibitor of blood glucose level elevation or the inhibitor of AGE generation of the present invention may comprise any other ingredients in addition to the acerola leaf extract and/or the processed product thereof.

The inhibitor of blood glucose level elevation or the inhibitor of AGE generation of the present invention can be in the form of a preparation comprising the acerola leaf extract and/or the processed product thereof in combination with a conventional pharmaceutical carrier. Dosage form is not particularly limited, and it is adequately determined according to need. In general, dosage forms can be oral preparations, such as tablets, capsules, granules, fine granules, powders, liquids, syrups, suspensions, emulsions, or elixirs or parenteral preparations, such as injections, drops, suppositories, inhalants, transdermal absorbents, transmucosal absorbents, adhesive preparations, or ointments.

Oral preparations are produced in accordance with conventional techniques using excipients, such as starch, lactose, sucrose, mannite, carboxymethylcellulose, cornstarch, or inorganic salt.

In addition to such excipients, this type of preparation can comprise, for example, a binder, a disintegrator, a surfactant, a lubricant, a flow promoter, a flavoring agent, a colorant, or a fragrant material.

Specific examples of binders include crystalline cellulose, crystalline cellulose carmellose sodium, methylcellulose, hydroxypropylcellulose, low-substituted hydroxypropylcellulose, hydroxypropylmethylcellulose, hydroxypropylmethylcellulose phthalate, hydroxypropylmethylcellulose acetate succinate, carmellose sodium, ethyl cellulose, carboxy methyl ethyl cellulose, hydroxyethyl cellulose, wheat starch, rice starch, cornstarch, potatostarch, dextrin, pregelatinized starch, partially pregelatinized starch, hydroxypropyl starch, Pullulan, polyvinylpyrrolidone, aminoalkyl methacrylate copolymer E, aminoalkyl methacrylate copolymer RS, mechacrylic acid copolymer L, mechacrylic acid copolymer, polyvinylacetal diethylaminoacetate, polyvinyl alcohol, gum Arabic, powdered acacia, agar, gelatin, white shellac, tragacanth, purified sucrose, and Macrogol.

Specific examples of disintegrators include crystalline cellulose, methylcellulose, low-substituted hydroxypropylcellulose, carmellose, carmellose calcium, carmellose sodium, croscarmellose sodium, wheat starch, rice starch, cornstarch, potatostarch, partially pregelatinized starch, hydroxypropyl starch, sodium carboxymethyl starch, and tragacanth.

Specific examples of surfactants include soybean lecithin, sucrose fatty acid ester, polyoxyl stearate, polyoxyethylene hydrogenated castor oil, polyoxyethylene polyoxypropylene glycol, sorbitan sesquioleate, sorbitan trioleate, sorbitan monostearate, sorbitan monopalmitate, sorbitan monolaurate, polysorbate, glyceryl monostearate, sodium lauryl sulfate, and lauromacrogol.

Specific examples of lubricants include wheat starch, rice starch, cornstarch, stearic acid, calcium stearate, magnesium stearate, hydrated silicon dioxide, light anhydrous silicic acid, synthetic aluminum silicate, dried aluminum hydroxide gel, talc, magnesium aluminometasilicate, calcium hydrogen phosphate, anhydrous dibasic calcium phosphate, sucrose fatty acid ester, waxes, hydrogenated vegetable oil, and polyethylene glycol.

Specific examples of flow promoters include hydrated silicon dioxide, light anhydrous silicic acid, dried aluminum hydroxide gel, synthetic aluminum silicate, and magnesium silicate.

When the inhibitor of blood glucose level elevation or the inhibitor of AGE generation of the present invention is administered in the form of a liquid, syrup, suspension, emulsion, or elixir, it may contain a taste and flavor corrigent or a colorant.

The present invention also relates to a food product comprising the aforementioned inhibitor of blood glucose level elevation or inhibitor of AGE generation. The food product of the present invention may be in any form, such as a confectionary (e.g., a candy, troche, jam, or chewing gum) or a beverage (e.g., a refreshing beverage such as tea or an alcoholic beverage), as well as in the form of a general "food product." The food product of the present invention can be prepared in the form of food for specified health uses (e.g., food for preventing diabetes or diabetic complications). The content of the inhibitor of blood glucose level elevation or the inhibitor of AGE generation in the food product is not particularly limited as long as desired effects are attained.

The present invention also relates to a food product comprising an acerola leaf extract and/or a processed product thereof. Up to the present, acerola leaves have not yet been used in a food product.

Hereafter, the present invention is described in greater detail with reference to the following examples, although the technical scope of the present invention is not limited to these examples.

### Example 1

Acerola leaves were homogenized, and 3x volume of distilled water was added thereto, followed by 1-hour extraction. This procedure was carried out twice, and the extract was centrifuged, filtered, lyophilized, and then diluted in distilled water again. The resulting solution was applied to the C18 cartridge columns (Sep-Pak Vac 35cc C18 cartridge columns, Waters), washed with distilled water, and eluted with a 0.2% TFA/methanol solution. The elution fraction was evaporated to dryness to obtain a purified extract.

Subsequently, the polyphenol content in the resulting purified extract was measured by the Folin-Denis method. Specifically, the purified extract was dissolved in distilled water to a concentration of 0.5 mg/ml, and 0.1 ml of the resulting solution, 2.9 ml of distilled water, and 0.5 ml of the Folin-Ciocalteu reagent (Merck) were mixed. The mixture was allowed to stand for 3 minutes, a 20% sodium carbonate solution was added thereto, the mixture was allowed to stand for an additional 60 minutes, and the absorbance at 650 nm was measured. The analytical curve was made using catechin as a standard reference material.

As a result, the polyphenol content of the purified extract was found to be 25%.

### Example 2

The purified acerola leaf extract prepared in Example 1 was subjected to measurement of α-glucosidase inhibitory activity (maltase inhibitory activity) in the following manner.

To a commercialized rat intestinal acetone powder, 9x volume of 56 mM maleate buffer (pH 6.0) was added, and the mixture was homogenized using a glass homogenizer. The resultant was centrifuged, the supernatant was recovered, and it was designated as a crude enzyme solution. A 20-fold dilution of the crude enzyme solution was used in the maltase reaction.

At the outset, 0.6 ml of 2 mg/ml sample solution was added to 0.6 ml of 2% maltose solution, the mixture was incubated at 37°C for 5 minutes, 0.6 ml of the crude enzyme solution was added thereto, and the reaction was carried out at 37°C for 120 minutes. The enzyme was deactivated by 10 minutes of heating in boiling water, centrifugation was carried out, and the glucose level in the supernatant was measured by HPLC.

HPLC conditions were as follows.
Column: Shim-pack CLC-NH2 columns (6.0 x 150 mm)
Column temperature: room temperature
Flow rate: 2 ml/min
Mobile phase: 75% acetonitrile
Detection: RI

Separately, the reaction was similarly carried out except that distilled water was used instead of the sample solution in an amount that was the same as that of the solution, the reaction product was designated as a control sample, and the glucose level thereof was similarly measured. The result of measurement of the control sample was designated as maltase inhibition of 0%.

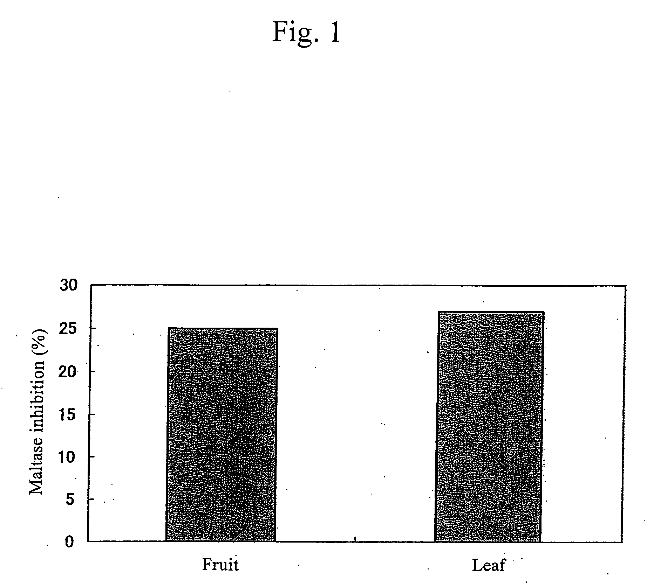

Fig. 1 shows the results of measurement by the above technique. Fig. 1 also shows the results of measurement of acerola fruit, which were obtained in the same manner as in the case of acerola leaf, as a comparative example.

As is apparent from Fig. 1, the purified acerola leaf extract has maltase inhibitory activity.

### Example 3

The purified acerola leaf extract prepared in Example 1 was subjected to measurement of inhibitory activity against AGE generation in the following manner.

Bovine serum albumin (16 mg/ml, 1 ml), 1 ml of 4M glucose, 1 ml of 1/15M phosphate buffer (pH 7.2), and 1 ml of 0.3 mg/ml sample solution were mixed, and the resultant was stored at 60°C. Seven days thereafter, AGE generated by a protein and a glucose was analyzed using a fluorescence spectrometer. The AGE initial product was analyzed using an excitation wavelength of 325 nm and an emission wavelength of 405 nm. The AGE advanced product was analyzed using an excitation wavelength of 370 nm and an emission wavelength of 440 nm. For the purpose of a comparison, an experiment involving the use of aminoguanidine was carried out in the same manner. Also, the same experiment was carried out except that distilled water was used instead of the sample solution in an amount that was the same as that of the solution, the reaction product was designated as a control sample, and the fluorescence analysis was similarly carried out. The result of measurement of the control sample was designated as AGE generation inhibition of 0%.

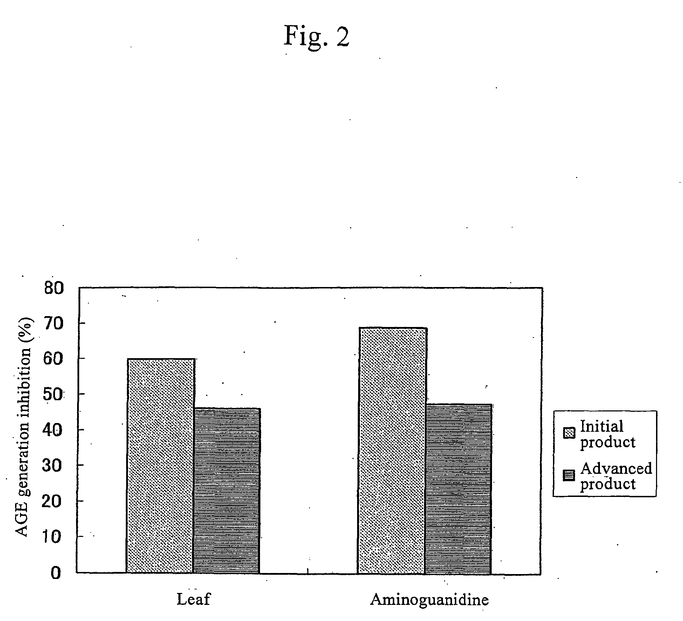

The results of measurement by the above technique are shown in Fig. 2.

As is apparent from Fig. 2, the purified acerola leaf extract has inhibitory activity against AGE generation that is equivalent to that of aminoguanidine.

All publications, patents, and patent applications cited herein are incorporated herein by reference in their entirety.

## Claims

1. An inhibitor of blood glucose level elevation comprising, as an active ingredient, an acerola leaf extract and/or a processed product thereof.

2. The inhibitor of blood glucose level elevation according to claim 1, wherein the acerola leaf extract and/or the processed product thereof comprise(s) polyphenols.

3. An inhibitor of advanced glycation end product (AGE) generation comprising, as an active ingredient, an acerola leaf extract and/or a processed product thereof.

4. The inhibitor of AGE generation according to claim 3, wherein the acerola leaf extract and/or the processed product thereof comprise(s) polyphenols.

5. The inhibitor of blood glucose level elevation or the inhibitor of AGE generation according to any one of claims 1 to 4, which is used to treat or prevent diabetes or diabetic complications.

6. The inhibitor of blood glucose level elevation or the inhibitor of AGE generation according to any one of claims 1 to 5, which is to be added to a food product.

7. A food product comprising the inhibitor of blood glucose level elevation or the inhibitor of AGE generation according to any one of claims 1 to 6.

8. A food product comprising an acerola leaf extract and/or a processed product thereof.
